# EUROPEAN PATENT APPLICATION

(11) **EP 2 796 452 A1**
(43) Date of publication of application: **29.10.2014**
(21) Application number: 13075030.0
(22) Date of filing: 23.04.2013
(51) Int. Cl.: C07D 303/08

(54) **Process for removal of 1,2-epoxy-5-hexene from epichlorohydrin**

(71) Applicant: Momentive Specialty Chemicals Research Belgium S.A., 1348 Ottignies Louvain-la-Neuve (BE)
(72) Inventor: Haesakkers, Paul, 1348 Ottignies Louvain-La-neuve (BE); Kapellen, Mark, 1348 Ottignies Louvain-La-neuve (BE); Lienke, Joachim, 1348 Ottignies Louvain-La-neuve (BE); Rens-Van der Lee, Sandra, 1348 Ottignies Louvain-La-neuve (BE); Van Rijn, Jimmy Antonius, 1348 Ottignies Louvain-La-neuve (BE)

(57) **Abstract**

The invention relates to a process for purification of epichlorohydrin containing 1,2-epoxy-5-hexene impurity, by
(a) epoxidizing allyl chloride contaminated with 1,5-hexadiene into epichlorohydrin,
(b) removing any unreacted allyl chloride,
(c) adding a halogen to the crude epichlorohydrin obtained after the removal of unreacted allyl chloride and allowing the halogen to react with 1,2-epoxy-5-hexene and other olefinically unsaturated components, if any, in the crude epichlorohydrin,
and
(d) rectifying the product of step (c) to obtain epichlorohydrin,
wherein the amount of halogen added in step (c) is at a molar ratio of at least 0.5:1 to less than 1:1 calculated on the amount of said 1,2-epoxy-5-hexene and said other olefinically unsaturated components in the crude epichlorohydrin, and allowing the halogen to react until it is fully converted.

## Description

### Technical field

This invention concerns a process for the removal of 1,2-epoxy-5-hexene from epichlorohydrin, in the preparation of epichlorohydrin by epoxidation of allyl chloride.

### Background art

Epichlorohydrin is commonly made by epoxidation of allyl chloride. Unfortunately, there is an impurity in the allyl chloride feedstock: 1,5-hexadiene. This impurity participates in the epoxidation and generates a side product 1,2-epoxy-5-hexene, which can cause problems in the further usage of the epichlorohydrin.

Rectification is the separation of the constituents of a liquid mixture by successive distillations (partial vaporizations and condensations) and is obtained via the use of an integral or differential process. Separations into effectively pure components may be obtained through this process (Perry's Chemical Engineers' Handbook, 6th edition, 18-3). Unfortunately, 1,2-epoxy-5-hexene has a boiling point of 118-121 °C and hence cannot be separated by rectification from epichlorohydrin (boiling temperature of 118°C). Therefore other means of removal are required.

CN 102417490 provides a process for purification of epichlorohydrin containing 1,2-epoxy-5-hexene. In this reference it was mentioned that epichlorohydrin can be obtained using conventional technology with a purity of 99.4% at most. This level of purity can be achieved by rectification. It was indicated that this may not be pure enough; a purity of 99.8% or more is desirable. To solve this problem, in the examples of this reference the contaminant 1,2-epoxy-5-hexene in the epichlorohydrin with a purity of 99.3% (in other words, rectified ECH) was treated with excess chlorine or bromine at a temperature in the range of 5 to 30°C. The 1,2-epoxy-5-hexene was then converted into a product with a significantly higher boiling temperature. The material in the reaction vessei was coloured, indicative of halogen remaining after reaction with the 1,2-epoxy-5-hexene or indeed with any other olefinically unsaturated component present in the epichlorohydrin. Next, remaining halogen was removed by flushing with nitrogen. Subsequently, rectification was used to obtain epichlorohydrin with a purity of more than 99.8%.

The process of CN'490 is an elegant process. On the other hand, it has some significant disadvantages. The reaction is carried out at temperatures that require active cooling of the epichlorohydrin, even below ambient temperatures. The reaction temperature according to this reference is 0-30°C, preferably 0-15°C, more preferably 0-5°C. It is mentioned that low temperatures are needed otherwise the excess of halogen results in by-product formation. Indeed, there is no teaching or suggestion that treatment with halogen would be possible without active cooling. However, cooling and in particular cooling below ambient temperatures adds complexity and costs.

Also, this reference specifically teaches that an excess of halogen has to be used (ratio halogen/1,2-epoxy-5-hexene between 1/1 and 3/1).

Moreover, after the reaction, the excess halogen must be removed (here by passing through nitrogen gas). This nitrogen stream needs to be treated to remove the halogen before being released into the atmosphere. Thus, there is a potential pollution issue with this process. This would again add complexity and costs.

The current inventors set out to remove 1,2-epoxy-5-hexene from epichlorohydrin without the aforementioned disadvantages.

### Disclosure of the invention

### Technical problem

Accordingly, the current invention provides a process as claimed in claim 1. More specifically, it provides a process for purification of epichlorohydrin containing 1,2-epoxy-5-hexene impurity, by
(a) epoxidizing allyl chloride contaminated with 1,5-hexadiene into epichlorohydrin,
(b) removing any unreacted allyl chloride,
(c) adding a halogen to the crude epichlorohydrin obtained after the removal of unreacted allyl chloride and allowing the halogen to react with 1,2-epoxy-5-hexene and other olefinically unsaturated components, if any, in the crude epichlorohydrin, and
(d) rectifying the product of step (c) to obtain epichlorohydrin, wherein the amount of halogen added in step (c) is at a molar ratio of at least 0.5:1 to less than 1:1 calculated on the amount of said 1,2-epoxy-5-hexene and said other olefinically unsaturated components in the crude epichlorohydrin, and allowing the halogen to react until it is fully converted.

### Technical solution

The current invention does not require an excess of halogen that has to be removed. Therefore no additional removal step such as a treatment with nitrogen flow is required. Moreover, the process according to the present invention can be performed at temperatures well above ambient temperature, up to the highest temperature of the rectification columns. In a preferred embodiment the invention does not require a chiller (according to Perry's Chemical Engineers' Handbook, 6th edition, 11-3: equipment that cools a fluid to a temperature below that obtainable if water only were used as coolant).

### Mode(s) for carrying out the invention

Preparation of epichlorohydrin by epoxidation is rather attractive. Of particular interest is the process for the manufacture of epichlorohydrin, in WO 2010/012360 by catalytic oxidation of allyl chloride with an oxidant wherein the catalytic oxidation is performed in an aqueous reaction medium, wherein a water-soluble manganese complex is used as oxidation catalyst, followed by the isolation of epichlorohydrin. The current invention, however, is not limited to this process. Other processes may be used as well, like processes having a titanium-based catalyst (WO2009115152) or tungsten-based catalyst (CN101016280).

The reaction is conducted at or above atmospheric pressure. The precise pressure is not critical so long as the reaction mixture is maintained substantially in a non-gaseous phase. Typical pressures vary from about 1 to about 100 atmospheres.

After the reaction, the crude epichlorohydrin is subjected to rectification steps, typically at elevated temperatures to remove unreacted allyl chloride, and other by-products (light ends which are all components with boiling points below epichlorohydrin, respectively heavy ends which are components with boiling points above epichlorohydrin). Temperature conditions up to 140 °C may be encountered during said rectification steps. In these subsequent steps it is advantageous to keep the epichlorohydrin at relatively elevated temperatures with little or no intermediate cooling. It is therefore the intent to avoid chillers that cool below the ambient temperature and the like.

Rectification conditions, such as distillation and fractional distillation, are known in the art.

Preferably, unreacted allyl chloride (for recycle purposes) and light ends, such as 1,5-hexadiene, chloropropanes and chloropropenes and the like, are removed, either together or separate, in one or more stages from the crude epichlorohydrin first. If separate, then the allyl chloride is removed first. The crude epichlorohydrin is subjected to distillation, preferably in a perforated-plate column, bubble-cap plate column and/or packed column. This may be a single column or a series of columns.

The column is preferably equipped with an evaporation or heating device (or an evaporation or heating area or zone) located at or near the bottom of the column (or below the first plate). It is furnished with means to introduce an inlet stream at a point intermediate between the bottom and the top of the column; means to withdraw a lower-boiling stream at or near the top of the column, and means to withdraw a higher-boiling stream at or near the bottom of the column and possibly means to withdraw a product stream at an intermediate point on the column.

Preferably, a lower-boiling stream is continuously drawn off at the head of the column and a higher-boiling stream is continuously drawn off at the foot of the column.

The process according to the invention is preferably performed as a continuous process.

Next, the heavy ends (e.g., components such as monochlorohydrins and dichlorohydrins and the like with boiling points greater than 118°C) are separated from epichlorohydrin. This is again done in a distillation column or series of columns, operating however at lower pressure and/or higher temperature conditions then the conditions for removal of the light ends. Moreover, the product stream is drawn off at the head of the column or at an intermediate point between feed point and the head of the column.

According to the invention, the 1,2-epoxy-5-hexene in the epichlorohydrin is converted into higher boiling products. This is done by using a reagent in the form of a halogen. Bromine and/or chlorine are most suitable to convert 1,2-epoxy-5-hexene into higher boiling products. Most preferably, chlorine, Cl₂, is used.

By ensuring that the 1,2-epoxy-5-hexene is converted into high boiling products, this contaminant can effectively be removed together with the heavy ends. In other words, it is not necessary to isolate nearly pure epichlorohydrin first and then convert the residual 1,2-epoxy-5-hexene, as is done in the CN'490 reference.

As indicated, if other olefinically unsaturated components in the epichlorohydrin are present, they will be converted at the same time. Thus, although the invention is mainly focussed on the removal of 1,2-epoxy-5-hexene, it can be used without difficulty to remove other contaminants, too. Contaminating amounts within the definition of the current invention are amounts in the range of about 0 to 25 % by weight of the allyl chloride feedstock, more preferably in the range of about 0 to 5 % by weight of the allyl chloride feedstock, still more preferably in the range of about 0 to 2 % by weight of the allyl chloride feedstock

Also, where CN'490 concerns a process at sub room temperatures, the process of the current invention rather converts 1,2-epoxy-5-hexene at elevated temperatures. This is beneficial since this removes the need for a chiller or similar sub-ambient cooler. Thus, CN'490 teaches in its examples to collect pure epichlorohydrin first, and then convert it into epichlorohydrin with improved purity. Effectively, this means that the epichlorohydrin is subjected to rectification for removal of the light ends, rectification for the heavy ends and allowed to cool. It is then treated with halogen. There is a further rectification to remove the products obtained by the halogenation of the unsaturated components, including 1,2-epoxy-5-hexene.

In the current process, there is only a single final rectification step wherein both the heavy ends and the products of the halogenation are effectively removed in a single step. Moreover, the epichlorohydrin, still at elevated temperature because of the removal of allyl chloride and light ends, may be used in the process of the current invention without intermediate cooling or with only mild cooling (e.g., if a heat exchanger is used, whereby hot epichlorohydrin exiting a distillation column is cooled by as much as 10 to100°C, preferably by as much as 30 to 80°C, whilst heating product that is sent to a distillation column, so as not to lose any heat). The epichlorohydrin may therefore be at a temperature anywhere between 30 and 140°C. If a heat exchanger is used, then the epichlorohydrin is preferably treated with halogen at about the temperature whereby it exits the heat exchanger. A chiller is not needed. This is a significant process advantage.

As indicated before, the epichlorohydrin may be treated immediately after the combined removal of allyl chloride and components with a boiling point below epichlorohydrin ("light ends"); after the removal of the allyl chloride and before removal of the light ends, which is the preferred embodiment, or after the removal of the light ends.

According to a preferred embodiment of the current invention, the 1,2-epoxy-5-hexene in the produced epichlorohydrin is converted in step (c) with a sub-stoichiometric amount of halogen, calculated on the amount of 1,2-epoxy-5-hexene and said olefinically unsaturated components in the epichlorohydrin, if any. Surprisingly good results are obtained at elevated temperatures with sub-stoichiometric amounts. For instance, good results have been achieved with halogen in an amount between 0.5:1 to less than 1:1, preferably 0.75:1 to 0.99:1, calculated on the1,2-epoxy-5-hexene and the olefinically unsaturated components in the crude epichlorohydrin. Moreover, this has the advantage that there is no need to remove the excess halogen. Removal of excess halogen with a nitrogen purge stream would be an environmental issue and adds complexity and costs.

Step (c) may be carried out at any stage after the removal of allyl chloride and before the final step (d). Indeed, if there are several columns used for the removal of the light and/or heavy ends, the reaction may be carried out between such columns, for instance up to a temperature within ± 10°C of the preceding distillation temperature.

Preferably, step (c) is carried out at a temperature in the range of 0 to 140 °C. Preferably the lower limit is at least 5 °C, more preferably above ambient temperature, more preferably above 30 °C. The upper temperature limit is preferably equal to about the highest temperature of the distillation columns. This could be a light ends distillation column or a heavy ends distillation column.

In a preferred embodiment of the current invention, the 1,2-epoxy-5-hexene in the produced epichlorohydrin is converted merely by combining the halogen with the epichlorohydrin stream that contains 1,2-epoxy-5-hexene. This may be a dedicated (additional) reactor, but also a simple mixing means may suffice.

It is preferred that the halogen (preferably chlorine) will be dissolved and well mixed or distributed into the mixture, especially because no excess halogen is used. Plug flow devices and/or CSTR devices can be used. A plug flow device (pipe reactor) can be combined with mixing elements, such as static mixer in order to enhance the mixing and equal distribution of the halogen. The halogen can be added at the entrance of the reactor device, but can also be staged and added at more locations. Addition of the halogen can be via nozzles or other devices.

Indeed preferably a static mixer is used with an inlet for the halogen upstream of the static mixer. This is the most preferred embodiment, wherein a halogen, preferably chlorine, is used at a temperature in the range of 25 to 140°C, downstream of the light ends column(s) and upstream of the heavy end column(s).

As an example for the most preferred embodiment, the dosing of the halogen is based on the analysis of the epichlorohydrin stream to prevent adding excessive amounts. The 1,2-epoxy-5-hexene content may be analysed. Alternatively, concentrations of the halogen dissolved in the epichlorohydrin product after addition and reaction can be analysed, for instance with spectroscopic methods, preferably UV-VIS spectroscopy. This can be performed both by analysis of samples taken from the stream or by direct in-line spectroscopic tools, located at least downstream to the dosing point(s) and mixing section of the halogen into the epichlorohydrin stream. The output of the analysis is then used to control the dosing of the halogen, thereby no excess halogen is used.

The following example illustrates the process of the invention and the use of apparatus according to the invention, but is not limitative thereof.

### EXAMPLES

Experimental set up. Model experiments were carried out in a jacketed glass reactor with approximately 250 ml reaction volume and with temperature control, reflux condenser, top stirrer and UV-VIS probe. Rather than first prepare epichlorohydrin and dose Cl₂ on the basis of the 1,2-epoxy-5-hexene contained therein, 1,2-epoxy-5-hexene is added to pure ECH containing a fixed amount of dissolved Cl₂. This has the advantage of reproducibility and facilitates comparison of the results.

The Cl₂ was dissolved in epichlorohydrin at 5 bar by using a Hastelloy autoclave and then transferred to the glass reactor. Cl₂ concentrations in epichlorohydrin are stable during transfer from autoclave to glass reactor. Concentrations of Cl₂ are measured by UV-VIS spectroscopy at a wavelength of 360 nm. Concentrations of 1,2-epoxy-5-hexene and epichlorohydrin are measured by gas chromatography. In a typical experiment, epichlorohydrin with dissolved Cl₂ was brought to a selected reaction temperature and then 1,2-epoxy-5-hexene was added to the reactor in different ratios to Cl₂. The mixture was stirred at 400 rpm.

### Comparative example C 1

To 250 ml epichlorohydrin with dissolved Cl₂ (31 mmol/l = 8.6 mmol) at 5 °C was added 1,2-epoxy-5-hexene (28 mmol/l = 7.8 mmol). After all 1,2-epoxy-5-hexene was reacted, 2.8 mmol/l (0.8 mmol) of Cl₂ remained in the epichlorohydrin. This reaction is described as entry 1 in Table 1. This experiment reflects prior art CN 102417490 , where an excess of Cl₂ over 1,2-epoxy-5-hexene was used and temperatures below 30 °C were required, preferably 5 °C. This reaction leads to residual Cl₂ in the reaction mixture, which is not desired. The low temperatures are undesired, because additional cooling below ambient temperature is required.

### Examples 2 to 7

The use of different temperatures and Cl₂/1,2-epoxy-5-hexene ratios and the results from the corresponding reactions are listed in Table 1 (entries 2-7). The reaction time to deplete the halogen, just like in comparative example C 1, is less than 5 seconds.

**Table 1. Chlorination reaction of 1,2-epoxy-5-hexene in epichlorohydrin**

| entry | Temperature (°C) | Cl₂ added (mmol/l) | 1,2-epoxy-5-hexene added (mmol/l) | Cl₂ molar ratio (*) | 1,2-epoxy-5-hexene remaining after reaction (mmol/l) | Cl₂ remaining after reaction (mmol/l) |
|---|---|---|---|---|---|---|
| C 1 | 5 | 30.9 | 28.1 | 1.1 | 0 | 2.8 |
| 2 | 5 | 14 | 24.9 | 0.6 | 10.7 | 0 |
| 3 | 5 | 11.4 | 25.1 | 0.5 | 13.6 | 0 |
| 4 | 60 | 18.7 | 27.5 | 0.7 | 8.7 | 0 |
| 5 | 60 | 14.7 | 28.7 | 0.5 | 14.0 | 0 |
| 6 | 100 | 29.4 | 32.6 | 0.9 | 3.3 | 0 |
| 7 | 100 | 17.2 | 21.4 | 0.8 | 1.5 | 0 |

| | | | | | | |
|---|---|---|---|---|---|---|
| (*) calculated on 1,2-epoxy-5-hexene and other olefinically unsaturated components in the epichlorohydrin | | | | | | |

The reaction performs well at temperatures above 30°C, were the Cl₂ reacts highly selective with 1,2-epoxy-5-hexene. Use of excess chlorine results in the full removal of 1,2-epoxy-5-hexene, but the presence of excess chlorine in the product. Comparison of experiments 1 (comparative), 2 and 3 reveal that significant amounts of 1,2-epoxy-5-hexene may be removed with substoichiometric amounts of chlorine. Experiments 5 to 7 illustrate that elevated temperatures may be used.

### Industrial applicability

Preparation of very pure epichlorohydrin without 1,2-epoxy-5-hexene contamination, and without use of excess halogens.

## Claims

1. A process for purification of epichlorohydrin containing 1,2-epoxy-5-hexene impurity, by
(a) epoxidizing allyl chloride contaminated with 1,5-hexadiene into epichlorohydrin,
(b) removing any unreacted allyl chloride,
(c) adding a halogen to the crude epichlorohydrin obtained after the removal of unreacted allyl chloride and allowing the halogen to react with 1,2-epoxy-5-hexene and other olefinically unsaturated components, if any, in the crude epichlorohydrin, and
(d) rectifying the product of step (c) to obtain epichlorohydrin,
wherein the amount of halogen added in step (c) is at a molar ratio of at least 0.5:1 to less than 1:1 calculated on the amount of said 1,2-epoxy-5-hexene and said other olefinically unsaturated components in the crude epichlorohydrin, and allowing the halogen to react until it is fully converted.

2. The process of claim 1, wherein
the amount of halogen added in step (c) is at a molar ratio of at least 0.75:1 to 0.99:1 calculated on the amount of said 1,2-epoxy-5-hexene and said olefinically unsaturated components in the crude epichlorohydrin.

3. The process of claim 1 or 2, wherein the reaction in step (c) is carried out at a temperature up to 140°C.

4. The process of claim 3, wherein the reaction in step (c) is carried out at a temperature greater than 30°C.

5. The process of claim 3 or 4, wherein unreacted allyl chloride (I) and components (II) with boiling points below epichlorohydrin ("light ends") are removed separately, with (I) being removed first, and with step (c) either before or after, preferably before the removal of (II).

6. The process of claim 5, wherein the reaction in step (c) is carried out at a temperature from 30°C up to the temperature corresponding with the preceding distillation temperature.

7. The process of any one of claims 1 to 6, by mixing in step (c) the added halogen with the crude epichlorohydrin obtained after the removal of unreacted allyl chloride using a mixing means.

8. The process of claim 7, wherein the mixing means is a static mixer.

9. The process of any one of claims 1 to 8, by dosing of the added halogen in an amount based on the analysis of the 1,2-epoxy-5-hexene and said olefinically unsaturated components in the crude epichlorohydrin or by analysing the residual halogen, if any, in the epichlorohydrin product of step (c).

10. The process of claim 9, by analysing with spectroscopic methods.

11. The process of claim 9, by analysing by direct in-line spectroscopic tools after adding and mixing halogen into the crude epichlorohydrin.
